# EUROPEAN PATENT SPECIFICATION

(11) **EP 1 263 504 B1**
(45) Date of publication and mention of the grant of the patent: **20.08.2003**
(21) Application number: 01918208.8
(22) Date of filing: 23.02.2001
(51) Int. Cl.: A61P 27/02, A61P 27/06, A61K 31/137, A61K 31/36, A61K 31/404, A61K 31/428, A61K 31/438, A61K 31/4433, A61K 31/4439, A61K 31/451, A61K 31/4545, A61K 31/445, A61K 31/4745, A61K 31/496, A61K 31/553

(54) **USE OF COMPOUNDS WITH 5-HT1A ACTIVITY USEFUL FOR TREATING DISORDERS OF THE OUTER RETINA**
VERWNDUNG VON VERBINDUNGEN MIT 5-HT1A AKTIVITÄT ZUR BEHANDLUNG VON KRANKHEITEN DER ÄUSSEREN RETINA
UTILISATION DE COMPOSES A ACTIVITE AGONISTE SUR LE RECEPTEUR 5-HT1A DESTINES AU TRAITEMENT DES AFFECTIONS DE LA RETINE EXTERNE

(30) Priority: 17.03.2000 US 190279 P
(43) Date of publication of application: 11.12.2002
(73) Proprietor: Alcon Inc., 6331 Hünenberg (CH)
(72) Inventor: COLLIER, Robert, J., Jr., Arlington, TX 76016 (US); KAPIN, Michael, A., Arlington, TX 76016 (US); HELLBERG, Mark, R., Highland Village TX 75077 (US); DEAN, Thomas, R., Weatherford, TX 76087 (US)
(74) Representative: Wain, Christopher Paul
(86) International application number: US0105700
(87) International publication number: WO01070222

(56) References cited:
- WO-A-98/18458
- WO-A-99/26621
- URBINA M ET AL: "5HT1A receptor agonist differentially increases cyclic AMP concentration in intact and lesioned goldfish retina. In vitro inhibition of outgrowth by forskolin." NEUROCHEMISTRY INTERNATIONAL, (1996 NOV) 29 (5) 453-60. , XP001059779
- LIMA L P MATUS ET AL: "Serotonin inhibits outgrowth of goldfish retina and impairs the trophic effect of taurine." JOURNAL OF NEUROSCIENCE RESEARCH, vol. 38, no. 4, 1994, pages 444-450, XP001059774 ISSN: 0360-4012
- LIMA L ET AL: "8-(3H)hydroxy-2-(di-n-propylamino)tetrali n binding sites in goldfish retina." NEUROCHEMICAL RESEARCH, vol. 19, no. 3, 1994, pages 249-255, XP001059781 ISSN: 0364-3190
- DATABASE WPI Section Ch, Week 199128 Derwent Publications Ltd., London, GB; Class B05, AN 1991-203975 XP002190916 & JP 03 128332 A (EISAI CO LTD), 31 May 1991 (1991-05-31)
- CHU T-C ET AL: "80H-DPAT-INDUCED OCULAR HYPOTENSION: SITES AND MECHANISMS OF ACTION" EXPERIMENTAL EYE RESEARCH, ACADEMIC PRESS LTD., LONDON, GB, vol. 69, no. 2, August 1999 (1999-08), pages 227-238, XP000879248 ISSN: 0014-4835
- DATABASE WPI Section Ch, Week 199049 Derwent Publications Ltd., London, GB; Class B03, AN 1990-364832 XP002190917 & JP 02 262518 A (KAKEN PHARM CO LTD), 25 October 1990 (1990-10-25)
- CHIDLOW G ET AL: "THE 5-HT1A RECEPTOR AGONIST 8-OH-DPAT LOWERS INTRAOCULAR PRESSURE IN NORMOTENSIVE NZW RABBITS" EXPERIMENTAL EYE RESEARCH, ACADEMIC PRESS LTD., LONDON, GB, vol. 69, no. 6, 1999, pages 587-593, XP000878788 ISSN: 0014-4835

## Description

The present invention is directed to the use of compounds with 5-HT_{1A} agonist activity for preparing a medicament for treating disorders of the outer retina resulting from acute or chronic degenerative conditions or diseases of the eye.

### Background of the Invention

Age-related macular degeneration (AMD) is the leading cause of blindness in the elderly, with an incidence of about 20% in adults 65 years of age increasing to 37% in individuals 75 years or older. Non-exudative AMD is characterized by drusen accumulation and atrophy of rod and cone photoreceptors in the outer retina, retinal pigment epithelium (RPE), Bruch's membrane and choriocapillaris; while exudative AMD leads to choroidal neovascularization (Green and Enger, *Ophthalmol*, 100:1519-35, 1993; Green et al., *Ophthalmol*, 92:615-27, 1985; Green and Key, *Trans Am Ophthalmol Soc*, 75:180-254, 1977; Bressler et al., *Retina*, 14:130-42, 1994; Schneider et al., *Retina,* 18:242-50, 1998; Green and Kuchle (1997). In: Yannuzzi, L.A., Flower, R.W., Slakter, J.S. (Eds.) *Indocyanine green angiography*. St. Louis: Mosby, p. 151-6). Retinitis pigmentosa (RP) represents a group of hereditary dystrophies characterized by rod degeneration with secondary atrophy of cone photoreceptors and underlying pigment epithelium. (Pruett, *Trans Am Ophthalmol Soc*, 81:693-735, 1983; Heckenlively, *Trans Am Ophthalmol Soc*, 85:438-470, 1987; Pagon, *Sur Ophthalmol*, 33:137-177, 1988; Berson, *Invest Ophthalmol Vis Sci*, 34:1659-1676, 1993; Nickells and Zack, *Ophthalmic Genet*, 17:145-65, 1996). The pathogenesis of retinal degenerative diseases such as AMD and RP is multifaceted and can be triggered by environmental factors in normal individuals or in those who are genetically predisposed. To date more than 100 genes have been mapped or cloned that may be associated with various outer retinal degenerations.

Light exposure is an environmental factor that has been identified as a contributing factor to the progression of retinal degenerative disorders such as AMD (Young, *Sur Ophthal*, 32:252-269, 1988; Taylor, et al., *Arch Ophthal*, 110:99-104, 1992; Cruickshank, et al., *Arch Ophthal*, 111:514-518, 1993). Photo-oxidative stress leading to light damage to retinal cells has been shown to be a useful model for studying retinal degenerative diseases for the following reasons: damage is primarily to the photoreceptors and retinal pigment epithelium (RPE) of the outer retina, the same cells that are affected in heredodegenerative diseases (Noell et al., *Invest Ophthal Vis Sci*, 5, 450-472, 1966; Bressler et al., *Sur Ophthal*, 32, 375-413, 1988; Curcio et al., *Invest Ophthal Vis Sci*, 37, 1236-1249, 1996); apoptosis is the cell death mechanism by which photoreceptor and RPE cells are lost in AMD and RP, as well as following a photo-oxidative induced cell injury (Ge-Zhi et at, *Trans AM Ophthal Soc*, 94, 411-430, 1996; Abler et al., *Res Commun Mol Pathol Pharmacol*, 92, 177-189, 1996; Nickells and Zack, *Ophthalmic Genet*, 17:145-65, 1996); light has been implicated as an environmental risk factor for progression of AMD and RP (Taylor et al., *Arch Ophthalmol*, 110, 99-104, 1992; Naash et al., *Invest Ophthal Vis Sci*, 37, 775-782, 1996); and therapeutic interventions which inhibit photo-oxidative injury have also been shown to be effective in animal models of heredodegenerative retinal disease (LaVail et al., *Proc Nat Acad Sci*, 89, 11249-11253, 1992; Fakforovich et al., *Nature*, 347, 83-86, 1990; Frasson et al., *Nat. Med*. 5, 1183-1187, 1990).

A number of different compound classes have been identified in various animal models that minimize retinal photo-oxidative injury. They include: antioxidants such as ascorbate (Organisciak et al., *Invest Ophthal Vis Sci*, 26:1589-1598, 1985), dimethylthiourea (Organisciak et al., *Invest Ophthal Vis Sci*, 33:1599-1609, 1992; Lam et al., *Arch Ophthal*, 108:1751-1752, 1990), α-tocopherol (Kozaki et al., *Nippon Ganka Gakkai Zasshi*, 98:948-954, 1994) and β-carotene (Rapp et al., *Cur Eye Res*, 15:219-232, 1995); calcium antagonists such as flunarizine (Li et al., *Exp Eye Res*, 56: 71-78, 1993; Edward et al., *Arch Ophthal*, 109, 554-622, 1992; Collier et al., *Invest Ophthal Vis Sci*, 36:S516); growth factors such as basic-fibroblast growth factor, brain derived nerve factor, ciliary neurotrophic factor, and interleukin-1-β (LaVail et al., *Proc Nat Acad Sci*, 89, 11249-11253, 1992); glucocorticoids such as methylprednisolone (Lam et al., *Graefes Arch Clin Exp Ophthal*, 231, 729-736, 1993) and dexamethasone (Fu et al., *Exp Eye Res,* 54, 583-594, 1992); iron chelators such as desferrioxamine (Li et al., *Cur Eye Res*, 2, 133-144, 1991); NMDA-antagonists such as eliprodil and MK-801 (Collier et al., *Invest Ophthal Vis Sci*, 40:S159, 1999).

Serotonergic 5-HT_{1A} agonists (i.e., buspirone, ziprasidone, urapidil) have either been registered or launched for the treatment of anxiety, hypertension, schidzophrenia, psychosis or depression-bipolar disorders. In addition, 5-HT_{1A} agonists have been shown to be neuroprotective in various animal models and are being evaluated in the clinic to treat cerebral ischemia, head trauma, Alzheimer's Disease, Multiple Sclerosis and amytrophic lateral sclerosis. The 5-HT_{1A} agonists, 8-OH-DPAT (8-hydroxy-2-(di-n-propylamino)tetralin) and ipsapirone, were shown to prevent NMDA-induced excitotoxic neuronal damage in the rat magnocellular nucleus basalis (Oosterink et al., *Eur J Pharmacol*, 358:147-52, 1998), dosing with Bay-x-3702 significantly reduced ischemic damage in a rat acute subdermal hematoma model (Alessandri et al., *Brain Res*, 845:232-5, 1999), while 8-OH-DPAT, Bay-x-3702, urapidil, gepirone and CM 57493 significantly reduced cortical infarct volume in the rat (Bielenberg and Burkhardt, *Stroke*, 21 (Supply: IV 161-3; Semkova et al., *Eur J Pharmacol*, 359:251-60, 1998; Peruche et al., *J Neural Transm - Park Dis Dement Sect*, 8:73-83, 1994) and mouse (Prehn et al., *Eur J Pharmacol*, 203:213-22, 1991; Prehn et al., *Brain Res*, 630:10-20, 1993) after occlusion of the middle cerebral artery. In addition, treatment of rats with SR 57746A, a potent 5-HT_{1A} agonist, has been shown to be neuroprotective following 4-vessel transient global ischernia, vincristine sulphate induced septohippocampal lesions, acrylamide-induced peripheral neuropathy, and sciatic nerve crush (Foumier et al., *Neurosci*, 55:629-41, 1993) and has been shown to delay the progression of motor neuron degeneration in *pmn* mice (Fournier et al., *Br J Pharmacol*, 124:811-7, 1998).

This class of compounds has been disclosed for the treatment of glaucoma (lowering and controlling IOP), see e.g., WO 98/18458 (DeSantis, et al) and EP 0771563A2 (Mano, et al.). Osborne, et al. (Ophthalmologica, Vol. 210:308-314, 1996) teach that 8-hydroxydipropylaminotetralin (8-OH-DPAT) (a 5-HT_{1A} agonist) reduces IOP in rabbits. Wang, et al. (Current Eye Research, Vol. 16(8):769-775, August 1997, and IVOS, Vol. 39(4), S488, March, 1998) disclose that 5-methylurapidil, an α_{1A} antagonist and 5-HT_{1A} agonist lowers IOP in the monkey, but due to its α_{1A} receptor activity. Also, 5-HT_{1A} antagonists are disclosed as being useful for the treatment of glaucoma (elevated IOP) (e.g. WO 92/0338, McLees). Furthermore, DeSai, et al. (WO 97/35579) and Macor, et al. (U.S. 5,578,612) disclose the use of 5-HT₁ and 5-HT₁₋ₗᵢₖₑ agonists for the treatment of glaucoma (elevated IOP). These anti-migraine compounds are 5-HT_{1B,D.E,F} agonists, e.g., sumatriptan and naratriptan and related compounds.

### Brief Description of the Drawings

**Figures 1A and 1B** show the preservation of the ERG a- and b-wave function in rats dosed systemically with 8-OH-DPAT and exposed to a severe photo-oxidative insult.
**Figure 2** shows protection of retinal morphology (photoreceptors and RPE) in rats dosed systemically with 8-OH-DPAT and exposed to a severe photo-oxidative insult.
**Figure 3** shows protection of retinal DNA, a measure of retinal cell number (A), and complete protection of retinal morphology (photoreceptors) in rats dosed systemically with buspirone and exposed to a severe photo-oxidative insult.
**Figure 4A and 4B** show the preservation of the ERG a- and b-wave function in rats dosed systemically with SR-57746A and exposed to a severe photo-oxidative insult.

### Summary of the Invention

The present invention is directed to the use of 5-HT_{1A} agonists for preparing a medicament useful in treating disorders of the outer retina, particularly: AMD; RP and other forms of heredodegenerative retinal disease; retinal detachment and tears; macular pucker; ischemia affecting the outer retina; diabetic retinopathy; damage associated with laser therapy (grid, focal, and panretinal) including photodynamic therapy (PDT); trauma; surgical (retinal translocation, subretinal surgery, or vitrectomy) or light-induced iatrogenic retinopathy; and preservation of retinal transplants.

### Description of Preferred Embodiments

Serotonergic 5-HT_{1A} agonists have been shown to be potent neuroprotective agents following varying insults to the central nervous system. Unexpectedly, we have demonstrated that 8-OH-DPAT (8-hydroxy-2-(di-n-propylamino)tetralin), buspirone and SR-57746A exhibit potent neuroprotective activity in the retina and prevent light-induced apoptotic cell death to photoreceptors and RPE cells. We have found that treatment with buspirone can completely prevent photo-oxidative induced retinopathy and significantly reduce loss of retinal DNA and ONL thinning. The safety advantages of some of these compounds make them particularly desirable for both acute and chronic therapies. Such an agent would have utility in the treatment of various outer retinal degenerative diseases.

In our light damage paradigms, antioxidants were either ineffective (α-tocopherol) or marginally effective at high doses (ascorbate, vitamin E analogs). Similarly, some calcium antagonists (flunarizine, nicardipine) were moderately effective while others (nifedipine, nimodipine, verapamil) had no effect in preventing light-induced functional or morphological changes. However, it has been discovered that 5-HT_{1A} agonists are 100-fold more potent in these light damage paradigms and therefore are useful for treating disorders of the outer retina.

The invention contemplates the use of any pharmaceutically acceptable 5-HT_{1A} agonist, including pharmaceutically acceptable salts, for preparing a medicament for treating disorders of the outer retina (Compounds). Pharmaceutically acceptable means the Compounds can be safely used for the treatment of diseases of the outer retina. As used herein, the outer retina includes the RPE, photoreceptors, Muller cells (to the extent that their processes extend into the outer retina), and the outer plexiform layer. The compounds are formulated for systemic or local ocular delivery.

Disorders of the outer retina encompass acute and chronic environmentally induced (trauma, ischemia, photo-oxidative stress) degenerative conditions of the photoreceptors and RPE cells in normal or genetically predisposed individuals. This would include, but not limited to, AMD, RP and other forms of heredodegenerative retinal disease, retinal detachment, tears, macular pucker, ischemia affecting the outer retina, diabetic retinopathy, damage associated with laser therapy (grid, focal and panretinal) including photodynamic therapy (PDT), thermal or cryotherapy, trauma, surgical (retinal translocation, subretinal surgery or vitrectomy) or light induced iatrogenic retinopathy and preservation of retinal transplants.

Compounds useful in the present invention have potent affinity for 5-HT_{1A} receptors with IC₅₀ values that range up to about 500 nM (preferably less than 100 nM). These Compounds are also either full or partial agonists with IC₅₀ values ranging up to about 1 µM (preferably less than 500 nM). Representative 5-HT_{1A} agonists useful according to the present invention include, but are not limited to: tandospirone, urapidil, ziprasidone, repinotan hydrochloride, xaliproden hydrochloride (SR-57746A), buspirone, flesinoxan, EMD-68843, DU-127090, gepirone, alnespirone, PNU-95666, AP-521, flibanserin, MKC-242, lesopitron, sarizotan hydrochloride, Org-13011, Org-12966, E-5842, SUN-N4057, and 8-OH-DPAT.

Receptor binding and agonist activity according to this invention can be determined using the following methods.

### METHOD 1

### 5-HT_{1A} Receptor Binding Assay

5-HT_{1A} binding studies were performed with human cloned receptors expressed in Chinese hamster ovary (CHO) cells using (³H)8-OH DPAT as the ligand. Membranes from Chinese hamster ovary cells (CHO) expressing cloned 5-HT_{1A} receptors (manufactured for NEN by Biosignal, Inc., Montreal, Canada) were homogenized in approximately 40 volumes of 50 mM Tris pH 7.4 for 5 sec. Drug dilutions were made using a Beckman Biomek 2000 robot (Beckman Instruments, Fullerton, CA). Incubations were conducted with membrane prep, test compounds, and 0.25 nM [³H]8-OH-DPAT (NEN, Boston, MA) in the same buffer at 27°C for 1 h. Assays were terminated by rapid vacuum filtration over Whatman GF/B glass fiber filters pre-soaked in 0.3% polyethyleneimine. Bound radioactivity was measured using liquid scintillation spectrometry. Data were analyzed using non-linear curve fitting programs (Sharif et al., J Pharmac Pharmacol, 51: 685-694, 1999).

Ligand binding studies can also be run using membrane preparations from calf and rat brain (local source) and human cortex membranes. Specific brain regions were dissected out, homogenized in 10 volumes of 0.32 M sucrose and centrifuged for 10 min at 700 x g. The resulting supernatant was centrifuged at 43,500 x g for 10 min and the pellet re-suspended in 50 mM Tris-HCl (pH 7.7, 25°C) using a 10 sec polytron treatment. Aliquots were stored at -140° C. To remove endogenous serotonin, the preps were incubated at 37° C for 10 min prior to the experiment. Assay incubations were terminated by rapid filtration over Whatman GF/C filters using a Brandel cell harvester. Kᵢ values were calculated using the Cheng-Prusoff equation (De Vry et al., J Pharm Exper Ther, 284:1082-1094, 1998.)

### METHOD 2

### 5-HT_{1A} Functional Assays

The function of Compounds of the present invention can be determined using a variety of methods to assess the functional activity of 5-HT_{1A} agonists. One such assay is performed using hippocampal slices from male Sprague-Dawley rats, measuring the inhibition of forskolin-stimated adenylate cyclase (J Med Chem, 42:36, 1999; J Neurochem, 56:1114, 1991; J Pharm Exper Ther, 284:1082, 1998). Rat hippocampal membranes were homogenized in 25 volumes of 0.3 M sucrose containing 1mM EGTA, 5 mM EDTA, 5 mM dithiothreitol, and 20 mM Tris-HCl, pH 7.4 at 25°C. The homogenate was centrifuged for 10 m in at 1,000 x *g*. The supernatant subsequently was centrifuged at 39,000 x g for 10 min. The resulting pellet was re-suspended in homogenization buffer at a protein concentration of approximately 1 mg/ml and aliquots were stored at -140°C. Prior to use, the membranes were rehomogenized in a Potter-Elvehjem homogenizer. Fifty µl of the membrane suspension (50 µg protein) were added to an incubation buffer containing 100 mM NaCI, 2 mM magnesium acetate, 0.2 mM ATP, 1 mM cAMP, 0.01 mM GTP, 0.01 mM forskolin, 80 mM Tris-HCl, 5 mM creatine phosphate, 0.8 U/µl creatine phosphokinase, 0.1 mM IBMX, 1-2 µCi α-[³²P]ATP. Incubations with test compounds (10 min at 30°C) were initiated by the addition of the membrane solution to the incubation mixture (prewarmed 5 min at 30°C). [³²P]cAMP was measured according to the method of Salomon (Adv Cyclic Nucleotide Res, 10:35-55, 1979). Protein was measured using the Bradford (Anal Biochem, 72:248-254, 1976) assay.

Functional activity can also be determined in recombinant human receptors according to the method of Schoeffter et al. (Neuropharm, 36:429-437, 1997). HeLa cells transfected with recombinant human 5-HT_{1A} receptors were grown to confluence in 24-well plates. The cells were rinsed with 1 ml of Hepes-buffered saline (in mM) NaCl 130, KCl 5.4, CaCl₂ 1.8, MgSO₄ 0.8, NaH₂PO₄ 0.9, glucose 25, Hepes 20, pH 7.4, and phenol red 5 mg/l. The cells were labelled with 6 µCi/ml of [³H]adenine (23 Ci/mmol, Amersham, Rahn AG, Zurich, Switzerland) in 0.5 ml of saline at 37 °C for 2 hr. The plates were subsequently rinsed twice with 1 ml of buffered saline containing 1mM isobutylmethylxanthine. The cells were incubated for 15 min in 1 ml of this solution (37 °C) in the presence or absence of 10 µM forskolin and the test compound. The buffer was then removed and 1 ml of 5% trichloroacetic acid (TCA) containing 0.1 mM cAMP and 0.1 mM ATP was added to extract the samples. After 30 min at 4°C, the TCA extracts were subjected to chromatographic separation on Dowex AG 50W-X4 and alumina columns (Salomon, Meth Enzymol, 195: 22-28, 1991). Cyclic AMP production was calculated as the ratio [³H]cAMP/([³H]cAMP + [³H]ATP).

The above procedures described in Methods 1 and 2 were used to generate the following data.

**Table 1.**

| **5-HT**_{**1A**} **Receptor Binding and Functional Assay Data.** | | |
|---|---|---|
| **Compound** | **Receptor Binding (IC**_{**50**} **nM, SEM)** | **cAMP Inhibition (EC**_{**50**}**)** |
| **(R,S) 8-OH-DPAT** | **1.5 nM** | **4.7 nM** |
| **(R) 8-OH-DPAT** | **0.5 nM** | **2.6 nM** |
| **SR-57746A** | **2.5 nM** | **3.7 nM** |

In general, for degenerative diseases, the 5-HT_{1A} agonists of this invention are administered orally with daily dosage of these compounds ranging between about 0.001 and about 500 milligrams. The preferred total daily dose ranges between about 1 and about 100 milligrams. Non-oral administration, such as, intravitreal, topical ocular, transdermal patch, subdermal, parenteral, intraocular, subconjunctival, or retrobulbar or subtenon's injection, trans scleral (including iontophoresis), or slow release biodegradable polymers or liposomes may require an adjustment of the total daily dose necessary to provide a therapeutically effective amount of the compound. The 5-HT_{1A} agonists can also be delivered in ocular irrigating solutions. Concentrations should range from about 0.001 µM to about 100 µM, preferably about 0.01 µM to about 5 µM.

The 5-HT_{1A} agonists can be incorporated into various types of ophthalmic formulations for delivery to the eye (e.g., topically, intracamerally, or via an implant). They may be combined with ophthalmologically acceptable preservatives, surfactants, viscosity enhancers, gelling agents, penetration enhancers, buffers, sodium chloride, and water to form aqueous, sterile ophthalmic suspensions or solutions or preformed gels or gels formed in situ. Ophthalmic solution formulations may be prepared by dissolving the compound in a physiologically acceptable isotonic aqueous buffer. Further, the ophthalmic solution may include an ophthalmologically acceptable surfactant to assist in dissolving the compound. The ophthalmic solutions may contain a viscosity enhancer, such as, hydroxymethylcellulose, hydroxyethylcellulose, hydroxypropylmethylcellulose, methylcellulose, polyvinyl-pyrrolidone, or the like, to improve the retention of the formulation in the conjunctival sac. In order to prepare sterile ophthalmic ointment formulations, the active ingredient is combined with a preservative in an appropriate vehicle, such as, mineral oil, liquid lanolin, or white petrolatum. Sterile ophthalmic gel formulations may be prepared by suspending the active ingredient in a hydrophilic base prepared from the combination of, for example, carbopol-940, or the like, according to the published formulations for analogous ophthalmic preparations; preservatives and tonicity agents can be incorporated.

If dosed topically, the 5-HT_{1A} agonists are preferably formulated as topical ophthalmic suspensions or solutions, with a pH of about 4 to 8. The 5-HT_{1A} agonists will normally be contained in these formulations in an amount .001% to 5% by weight, but preferably in an amount of .01% to 2% by weight. Thus, for topical presentation, 1 to 2 drops of these formulations would be delivered to the surface of the eye 1 to 4 times per day according to the discretion of a skilled clinician.

The following topical ophthalmaic formulations are useful according to the present invention administered 1-4 times per day according to the discretion of a skilled clinician.

**EXAMPLE 1**

| **Ingredients** | **Amount (wt %)** |
|---|---|
| Buspirone | 0.01 - 2% |
| Hydroxypropyl methylcellulose | 0.5% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

**EXAMPLE 2**

| **Ingredients** | **Amount (wt %)** |
|---|---|
| Buspirone | 0.01 - 2% |
| Methyl cellulose | 4.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

**EXAMPLE 3**

| **Ingredients** | **Amount (wt %)** |
|---|---|
| Compound | 0.01 - 2% |
| Guar gum | 0.4- 6.0% |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |
| Purified water | q.s. to 100% |

**EXAMPLE 4**

| **Ingredients** | **Amount (wt %)** |
|---|---|
| Xaliproden hydrochloride | 0.01 - 2% |
| White petrolatum and mineral oil and lanolin | Ointment consistency |
| Dibasic sodium phosphate (anhydrous) | 0.2% |
| Sodium chloride | 0.5% |
| Disodium EDTA (Edetate disodium) | 0.01% |
| Polysorbate 80 | 0.05% |
| Benzalkonium chloride | 0.01% |
| Sodium hydroxide / Hydrochloric acid | For adjusting pH to 7.3 - 7.4 |

**EXAMPLE 5**

| **10mM IV Solution** | **w/v%** |
|---|---|
| Buspirone | 0.384% |
| L-Tartaric acid | 2.31% |
| Sodium hydroxide | pH 3.8 |
| Hydrochloric acid | pH 3.8 |
| Purified water | q.s. 100% |

**EXAMPLE 6**

| **5mg Capsules** | |
|---|---|
| **Ingredient** | **mg/capsule (Total Wt. 22a mg)** |
| Buspirone Hydrochloride | 5 |
| Lactose, anhydrous | 55.7 |
| Strach, Sodium carboxy-methyl | 8 |
| Cellulose, microcrystalline | 30 |
| Colloidal silicon dioxide | .5 |
| Magnesium sterage | .8 |

### METHOD 3

### Neuroprotective effects in the rat photo-oxidative induced retinopathy model

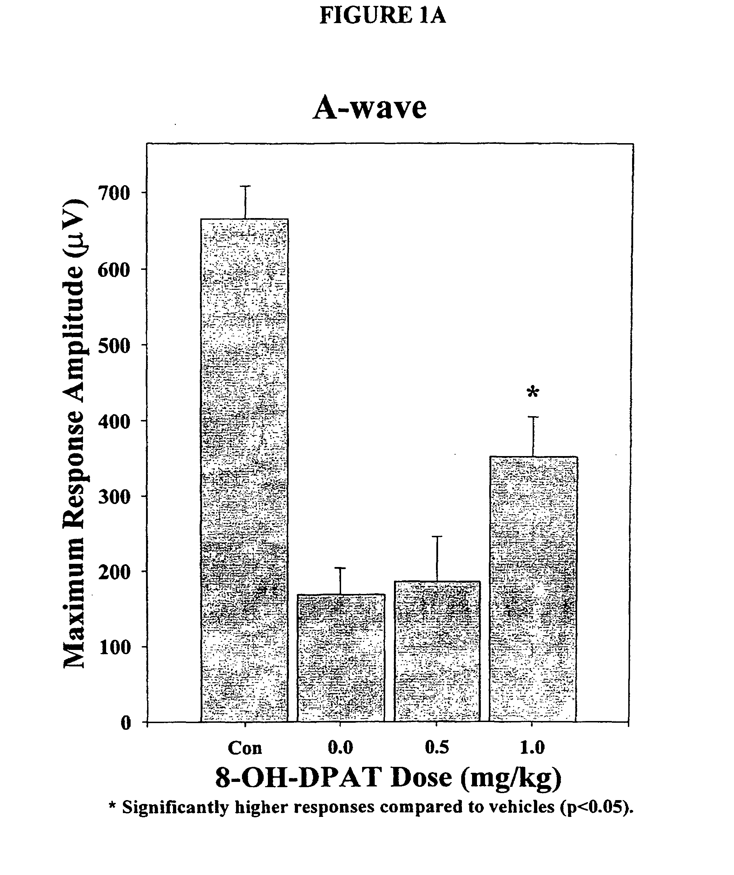

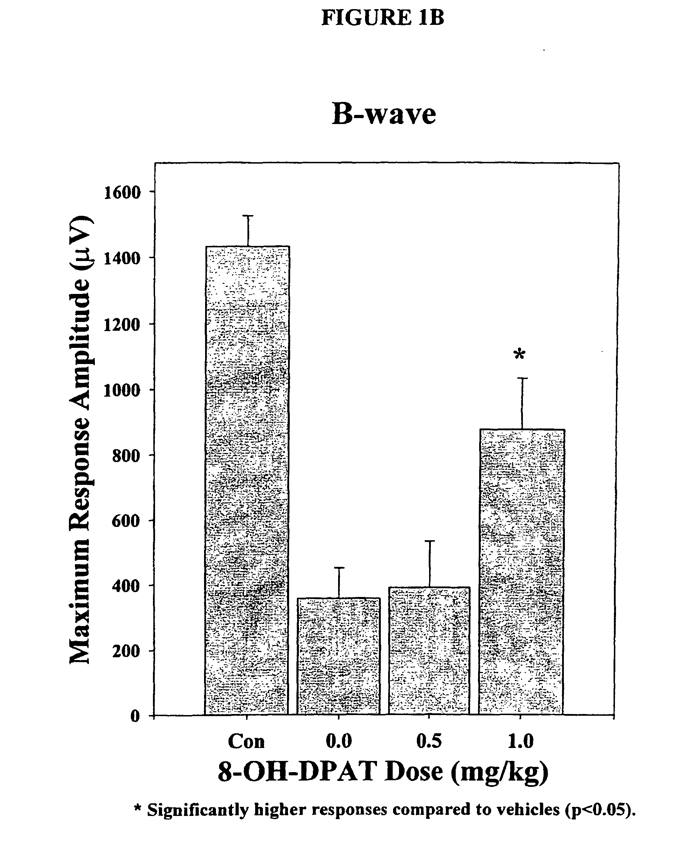

The retinal protective effect of these 5-HT_{1A} agonists were evaluated in our photo-oxidative induced retinopathy paradigm.
**Induction of Photochemical Lesion.** Photochemical lesions were induced in dark adapted rats (24 hour) by exposure to (220 fc) blue light (half-amplitude bandpass = 435-475 nm) for 6 hours. Animals were allowed to recover for 5 days in darkness prior to electrodiagnostic evaluation of retinal function. Rats were single housed in clear polycarbonate cages during this light exposure.
**Electrodiagnostic Evaluation.** The electroretinogram (ERG) was recorded from anesthetized rats after a 24-hour dark-adaptation period. Rats were anesthetized by IP injection with Ketamine-HCl (75 mg/Kg) and Xylazine (6 mg/Kg). Flash ERGs recorded from a platinum-iridium wire loop electrode positioned on the cornea were elicited by viewing a ganzfeld. Electrical responses to a series of light flashes increasing in intensity were digitized to analyze temporal characteristics of the waveform and response voltage-log intensity (VlogI) relationship. Changes in the ERG a-wave are associated with photoreceptor and retinal pigment epithelium damage while damage to the inner retina is reflected in changes in the ERG b-wave.
**Assessment of Retinal Morphology.** Ocular tissues were obtained from control and drug or vehicle dosed rats and fixed by immersion into a mixture of 2% paraformaldehyde and 2% glutaraldehyde. Fixed eyeballs were dehydrated in an ascending ethanol series, embedded in JB-4 plastic resin, and 1 to 1.5-micron thick sections were analyzed using a quantitative computer image analysis system attached to the microscope. Retinal layer thickness (retinal pigment epithelium, RPE; outer nuclear layer thickness, ONL; inner nuclear layer thickness, INL; and length of photoreceptor inner and outer segments, IS+OS) was measured.
**Assessment of DNA Changes.** Albino rats were euthanized by CO₂ inhalation and individual retinas were frozen in separate tubes. Each retina had been sonicated in 0.8 ml (2.0 M NaCl, 50 mM NaPO₄, pH 7.4, 2 mM EDTA) to yield a uniform homogenate, and stored frozen. Aliquots (0.1 ml) of each sample were diluted 10-fold with 2.0 M NaCl, 50 mM NaPO₄. pH 7.4, 2 mM EDTA containing 1.1 µg/ml bisbenzimidazole (Hoechst 33258). A standard curve was constructed using calf thymus DNA from 0 to 25 µg/ml in the same buffer. Triplicate 0.2 ml aliquots of each retina sample and standard were pipetted into a 96 well plate for fluorescence measurements in the Cytofluor II. The excitation wavelength was 360 nm, and the emission wavelength was 460 nm.
**Subjects and Dosing.** Male Sprague Dawley rats were randomly assigned to drug and vehicle experimental groups. Control rats were housed in their home cage under normal cyclic light exposure. All rats were dosed 48, 24 and 0 hours prior to a 6-hour blue-light exposure. Dosing was as follows:
**1.) *8-OH-DPAT* (8-hydroxy-2-(di-n-propylamino)tetralin):** Rats receiving either vehicle (N=10) or 8-OH-DPAT (0.5 mg/kg [N=5] or 1.0 mg/kg [N=10]) were given three subcutaneous (SC) injections prior to light exposure. Five rats were used as controls. Retinal protection was assessed by analyzing the ERG response and measuring changes in retinal morphology.
**2.) *Buspironei*:** For DNA quantitation, six rats per treatment group were dosed IP with vehicle or buspirone (0.5 and 1 mg/kg) prior to light exposure. Retinas from seven normal rats were used as controls. To evaluate changes in retinal morphology, rats were dosed (IP) with either vehicle (N=8) or buspirone (1.0 mg/kg [N=9]). Six rats were used as controls. Retinal protection was assessed by quantitating changes in retinal DNA and measuring changes in retinal morphology.
**3.) *SR-57746A*:** Rats were dosed (IP) with vehicle (N=15) or SR-57746A (0.5 mg/kg [N=5] or 1 mg/kg [N=15]). Eleven rats were used as controls. The ERG was analyzed after a 5-day recovery period to assess retinal protection.
**8-OH-DPAT Evaluation Results.** Blue-light exposure for 6 hours resulted in a significant diminution of the ERG response amplitude (ANOVA, p < 0.001; Bonferroni t-test, p < 0.05) compared to normals when measured after a 5-day recovery period (Figures 1A and B). Blue-light exposure resulted in a 75% reduction in the maximum a- and b-wave amplitudes in vehicle dosed rats compared to controls. In addition, threshold responses were lower and evoked at brighter flash intensities.

Rats dosed with 8-OH-DPAT showed dose-dependent protection of outer and inner retina function against this photo-oxidative induced retinopathy (Figures 1A and B). Maximum a- and b-wave response amplitudes in 8-OH-DPAT (0.5 mg/kg) dosed rats were not different than vehicle dosed rats and were approximately 27% of control amplitudes. However, maximum a- and b-wave response amplitudes from 8-OH-DPAT (1.0 mg/kg) dosed rats were approximately 53% and 61% of normal, respectively, and significantly higher than responses measured in vehicle dosed rats (Figures 1A and 1B).

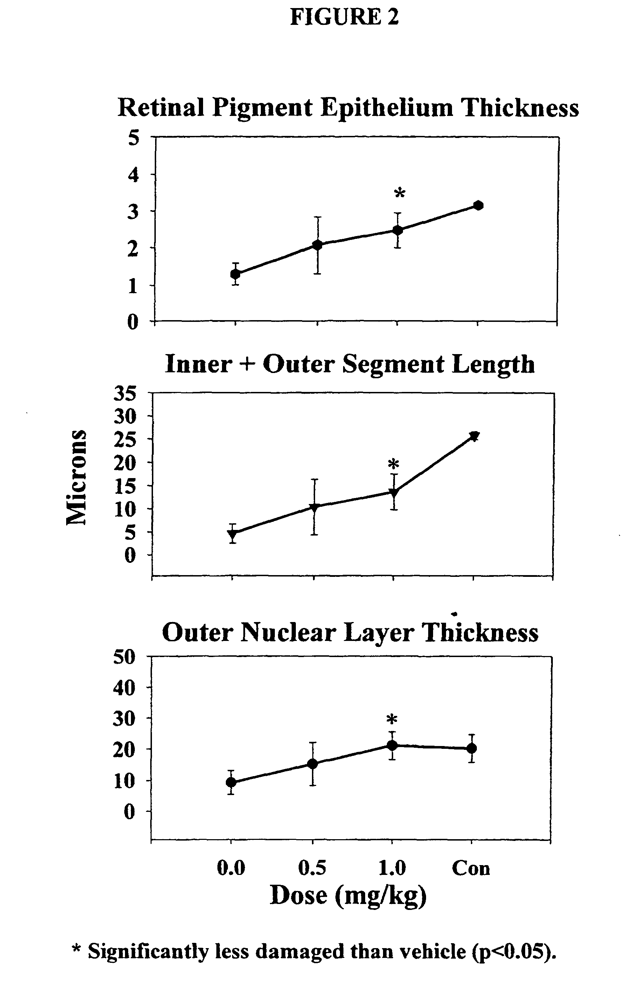

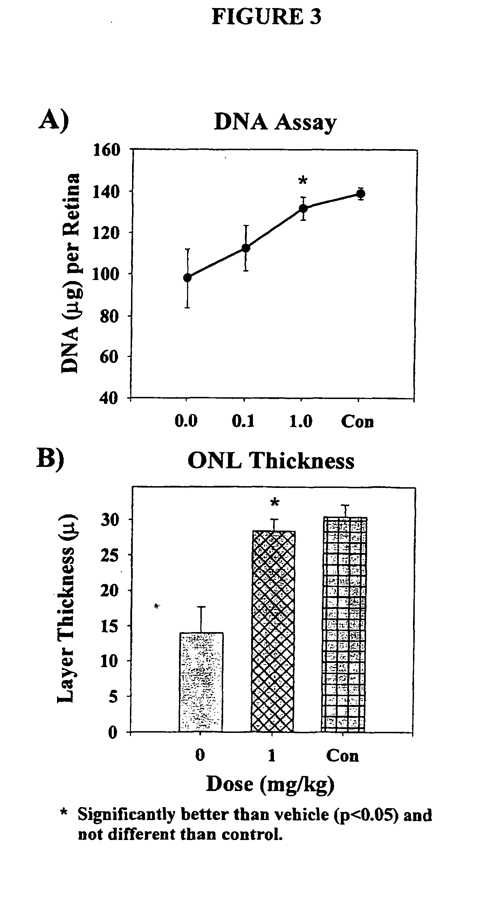

Consistent with these ERG changes, morphometric analysis of these retinas after a 3-week recovery period demonstrated a significant (ANOVA, p<0.01) loss of photoreceptor cells, shortening of photoreceptor inner + outer segment length, and flattening of the RPE in vehicle dosed animals. No significant changes in the thickness of the INL were detected. ONL thickness was reduced 73%, inner + outer segment length was reduced 82%, and RPE thickness was reduced 59% compared to controls (Figure 2). Lesions observed in rats dosed with 8-OH-DPAT (0.5 mg/kg) were not significantly different than lesions measured in vehicle dosed rats. While ERGs were reduced approximately 63%, the ONL thickness was reduced by 53%, photoreceptor segment length was reduced 60%, and the RPE thickness was reduced 34%. However, photic lesions observed in rats dosed with 8-OH-DPAT (1.0 mg/kg) were significantly different from vehicle dosed rats. While ERG response amplitudes were greater than 50% of normal, the ONL thickness was 2.4 fold thicker, photoreceptor segment length was 2.9 times longer, and RPE thickness was 1.9 times thicker compared to vehicle dosed rats.
**Buspirone Evaluation Results.** As seen in Figure 3A, vehicle dosed retinal DNA levels were significantly reduced (ANOVA, p=0.017) about 30% from control levels. No significant differences were measured between groups dosed with vehicle or 0.1 mg/kg buspirone. Retinal protection was measured in rats dosed with buspirone (1 mg/kg). Retinal DNA levels were significantly higher than measured in vehicle dosed rats, but not significantly different than controls.

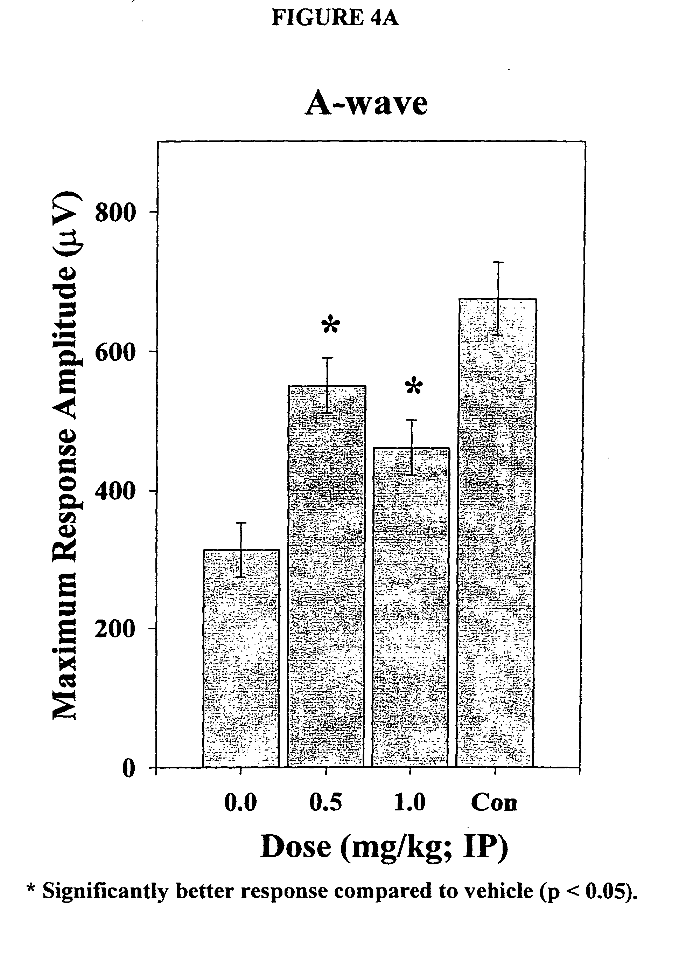

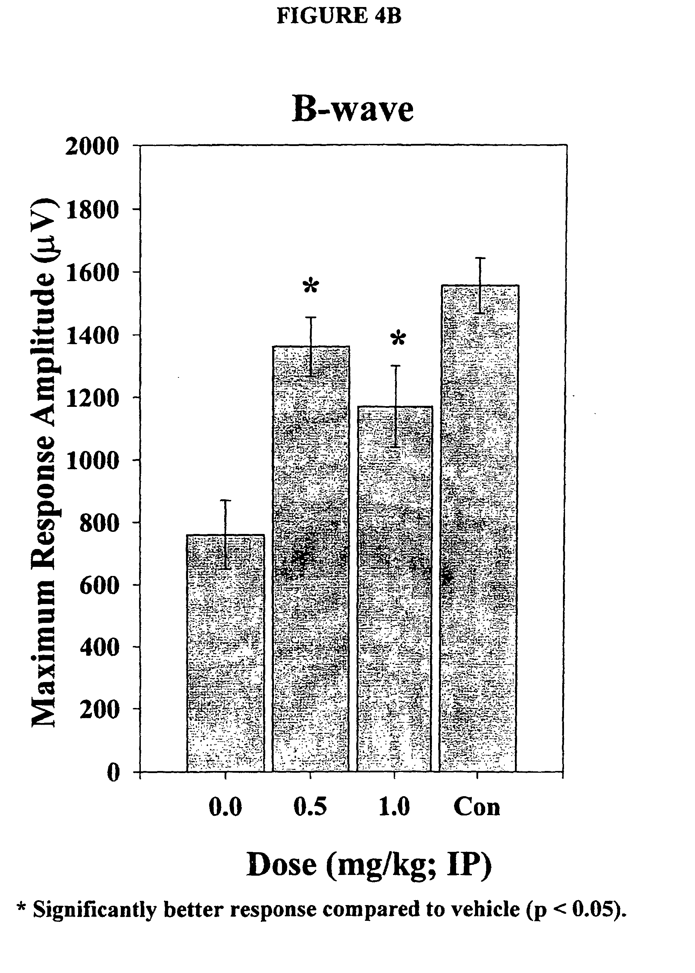

Blue-light exposure for 6 hours resulted in a significant reduction in photoreceptor number (ANOVA, p<0.05). Morphometric analysis of these retinas after a 4-week recovery period demonstrated a 54% thinning of the outer nuclear layer in vehicle dosed rats compared to controls (Figure 3B). However, no significant difference in ONL thickness was measured between normal and buspirone treated rats. In rats dosed with buspirone (1 mg/kg) the ONL thickness was 28.3 µ compared to 30.4 µ in normal rats.
**SR-57746A Evaluation Results.** Significant protection of retinal function was measured in light-exposed rats dosed with SR-57746A ( 0.5 and 1.0 mg/kg). Maximum a- and b-wave response amplitudes were reduced by 50% in vehicle dosed rats compared to controls (Figures 4A and B). Maximum responses were 82% of controls in rats dosed with SR-57746A (0.5 mg/kg) and 70% of normal in rats dosed with 1 mg/kg.
***Conclusion.*** These 5-HT_{1A} agonists (8-OH-DPAT, buspirone, and SR-57746A) demonstrated good potency and efficacy in this oxidative model of retinal degenerative disease. Functional and structural protection were achieved in rats dosed on three consecutive days with a dose as low as 1 mg/kg.

## Claims

1. The use of a compound with 5-HT_{1A} agonist activity for the preparation of a pharmaceutical composition for the treatment of disorders of the outer retina.

2. The use according to claim 1, of a compound selected from tandospirone, urapidil, ziprasidone, repinotan hydrochloride, xaliproden hydrochloride (SR-57746A), buspirone, flesinoxan, EMD-68843, DU-127090, gepirone, alnespirone, PNU-95666, AP-521, flibanserin, MKC-242, lesopitron, sarizotan hydrochloride, E-5842, SUN-N4057, Org-13011, Org-12966 and 8-OH-DPAT.

3. The use according to claim 1 or 2, wherein the disorder is selected from AMD; RP and other forms of heredodegenerative retinal disease; retinal detachment and tears; macular pucker, ischemia affecting the outer retina; diabetic retinopathy; damage associated with laser therapy (grid, focal, and panretinal) including photodynamic therapy (PDT); trauma; surgical (retinal translocation, subretinal surgery, or vitrectomy) or light-induced iatrogenic retinopathy; and preservation of retinal transplants.

4. The use according to claim 2, wherein the disorder is AMD, RP, or diabetic retinopathy.

## Patentansprüche

1. Verwendung einer eine 5-HT_{1A}-Antagonisten-Aktivität aufweisenden Verbindung für die Zubereitung einer pharmazeutischen Zusammensetzung für die Behandlung von krankhaften Störungen der äußeren Netzhaut.

2. Verwendung nach Anspruch 1 einer Verbindung, die unter Tandospiron, Urapidil, Ziprasidon, Repinotanhydrochlorid, Xaliprodenhydrochlorid (SR-57746A), Buspiron, Flesinoxan, EMD-68843, DU-127090, Gepiron, Alnespiron, PNU-95666, AP-521, Flibanserin, MKC-242, Lesopitron, Sarizotanhydrochlorid, E-5842, SUN-N4057, Org-13011, Org-12966 und 8-OH-DPAT ausgewählt wird.

3. Verwendung nach Anspruch 1 oder 2, wobei die krankhafte Störung unter AMD, RP und anderen Formen heredodegenerativer Netzhauterkrankungen, Netzhautloslösung und -rissen, epiretinaler Gliose, die äußere Netzhaut beeintträchtigender Ischämie, diabetischer Retinopathie, mit Lasertherapie verbundener Verletzung (Netzwerk-, Fokal- und Panretinaverletzung) einschließlich photodynamischer Therapie (PDT), Trauma, chirurgischen Eingriffen (Netzhautverpflanzung, subretinalen chirurgischen Eingriffen oder Vitrektomie) oder lichtinduzierter iatrogener Retinopathie und dem Konservieren von Netzhauttransplantaten ausgewählt wird.

4. Verwendung nach Anspruch 2, wobei die krankhafte Störung AMD, RP oder diabetische Retinopathie ist.

## Revendications

1. Utilisation d'un composé avec activité agoniste 5-HT_{1A} pour la préparation d'une composition pharmaceutique pour le traitement de désordres de la rétine externe.

2. Utilisation selon la revendication 1, d'un composé sélectionné parmi le tandospirone, l'urapidile, le ziprasidone, l'hydrochlorure de répinotane, l'hydrochlorure de xaliprodène (SR-57746A), le buspirone, le flésinoxane, l'EMD-68843, le DU-127090, le gépirone, l'alnéspirone, le PNU-95666, l'AP-521, la flibansérine, le MKC-242, le lésopitrone, l'hydrochlorure de sarozotan, l'E-5842, le SUN-N4057, l'Org-13011, l'Org-12966 et 1' 8-OH-DPAT.

3. Utilisation selon la revendication 1 ou 2, où le trouble est sélectionné parmi l'AMD; la RP et autres formes de maladie rétinienne hérédodégénérative ; le détachement de la rétine et larmes ; le plissement maculaire, l'ischémie affectant la rétine externe ; la rétinopathie diabétique ; les troubles associés à la thérapie laser (grille, focale et panrétinienne) englobant la thérapie photodynamique (PDT) ; le traumatisme ; la rétinopathie chirurgicale (translocation rétinienne, chirurgie subrétinienne ou vitrectomie) ou la rétinopathie iatrogénique induite par la lumière ; et la conservation des implants rétiniens.

4. Utilisation selon la revendication 2, dans laquelle le trouble est l'AMD, la RP ou la rétinopathie diabétique.
